Europäisches Patentamt

European Patent Office

Office européen des brevets

(11) Publication number: **0 265 111 B1**

## EUROPEAN PATENT SPECIFICATION

(45) Date of publication of patent specification: **18.03.92**   (51) Int. Cl.5: **C13K 13/00**

(21) Application number: **87308831.4**

(22) Date of filing: **06.10.87**

The file contains technical information submitted after the application was filed and not included in this specification

(54) **Process for the production of xylose.**

(30) Priority: **20.10.86 GB 8625095**

(43) Date of publication of application:
**27.04.88 Bulletin 88/17**

(45) Publication of the grant of the patent:
**18.03.92 Bulletin 92/12**

(84) Designated Contracting States:
**AT DE ES FR GB IT NL SE**

(56) References cited:
**EP-A- 0 178 777**
**FR-A- 1 514 931**
**US-A- 3 780 017**
**US-A- 3 784 408**

(73) Proprietor: **IMPERIAL CHEMICAL INDUSTRIES PLC**
**Imperial Chemical House, Millbank**
**London SW1P 3JF(GB)**

(72) Inventor: **Fields, Peter Richard**
**32 Leicester Way Eaglescliffe**
**Stockton-on-Tees Cleveland(GB)**
Inventor: **Wilson, Robin James**
**49 Newlands Avenue Norton**
**Stockton-on-Tees Cleveland(GB)**

(74) Representative: **Locke, Timothy John et al**
**Imperial Chemical Industries PLC Legal Department: Patents Bessemer Road PO Box 6 Welwyn Garden City Hertfordshire, AL7 1HD(GB)**

Rank Xerox (UK) Business Services

**Description**

This invention relates to a process for the production of xylose.

Xylose is a pentose sugar which is used in the production of xylitol and other sweetening additives for foods. It is prepared by the acid hydrolysis of ligno-cellulosic materials containing hemicelluloses having a high proportion of xylose units or xylans in their molecules. In addition to hemicelluloses, ligno-cellulosic materials contain lignin, cellulose and other carbohydrates. Furthermore hemicellulose molecules contain, in addition to xylans, units of other sugars. Hemicelluloses derived from different ligno-cellulosic materials vary in structure, some containing a greater proportion of xylans in their molecules than are contained in the molecules of other hemicelluloses. In the production of xylose it is necessary to separate lignin, cellulose and other materials from the product of the initial hydrolysis. Known processes for the production of xylose from ligno-cellulosic materials include that proposed in US Patent 3784408 which includes aqueous acid hydrolysis of cellulose and xylan containing materials, separation of precipitated lignins, treatment with an ion exchange resin, drying, dissolution in methanol and a final step in which xylose is recovered as a crystalline precipitate from a methanolic solution. US Patent 3780017 describes a method of recovering crystalline xylose from an impure aqueous solution including adding a water miscible alcohol such as ethanol, to the solution to precipitate impurities, removing the precipitated impurities, evaporating off the ethanol and crystallising the xylose by adding acetic acid to a concentration of at least 25%.

In the production of xylose from ligno-cellulosic materials on a commercial scale it is important that as little as possible of the xylose available in the starting materials is lost during separation from the other products of hydrolysis. Generally in processes previously used to produce xylose, separation has only been achieved with great difficulty and high losses. This has considerably limited the commercial value of the processes generally used to date.

According to the present invention we provide a process for the production of xylose from a ligno-cellulosic material comprising a xylan-containing hemicellulose, which comprises the steps of:-

(1) hydrolysing the ligno-cellulosic material with an aqueous acid to produce an aqueous xylose-containing medium and an insoluble residue comprising lignin and cellulose;

(2) separating the insoluble residue from the xylose-containing medium;

(3) treating the xylose-containing medium to remove organic and/or ionic contaminants therefrom;

(4) concentrating the xylose-containing medium to a syrup having a water content in the range 20% to 40% by weight; and

(5) separating the xylose from the syrup by crystallization characterised in that the separation of xylose is carried out by mixing the syrup with ethanol to form an ethanolic solution and crystallizing xylose from the ethanolic solution.

Preferably the crystals produced in step (5) are washed with aqueous ethanol in order to improve purity and to meet further processing criteria. Most suitably the aqueous ethanol is an ethanol/water mixture containing 90% ethanol.

After either step (2) or step (3) a post hydrolysis step may be performed to remove or reduce any oligosaccharides present. The reaction described in step (1) can be carried out in batch or continuous mode. If it is carried in continuous mode then the residue time distribution of the continuous reactor could give rise to incomplete hydrolysis of the hemicellulose to sugar monomers. The presence of hemicellulosic oligosaccharides can lead to problems during the subsequent crystalisation of xylose. Thus, to reduce this problem, a post-hydrolysis step may be employed to remove or reduce the oligosaccharide content. This would take the form of heating the xylose containing liquor to a temperature of between 50-150°C for a period within the range of 2 mins to 2 hours in the presence of a dilute acid of 0.5 to 5% in concentration. This would take place in a reactor with a discrete residence time such as a plug flow or batch reactor. In a commercial process a routine analysis can determine the amount of oligosaccharides present in order that it can be decided if post hydrolysis is required.

Suitably the ligno-cellulosic material is one containing hemicelluloses having a high proportion of xylans in their molecules. Examples of suitable materials include wheat straw, corn stover and bagasse.

The hydrolysis step (1) is suitably carried out using a dilute aqueous solution of an acid. Preferably a solution containing 0.5% to 5% acid in water is used. The acid may be an inorganic acid such as sulphuric acid, hydrochloric acid or phosphoric acid or an organic acid such as acetic acid or trifluoracetic acid. During hydrolysis the reaction mixture is suitably maintained at a temperature within the range 70° to 270°C, temperatures within the range 100° to 150°C being preferred. Hydrolysis is preferably carried out for a period within the range 2 minutes to 2 hours. For the hydrolysis step a suitable combination of process parameters is selected. For instance when higher temperatures are used, hydrolysis is preferably carried out using weaker solutions of acids and/or for shorter periods of time. Combinations of parameters at the upper ends of the suitable ranges, i.e. high temperatures for longer periods using stronger solutions of acids, are not preferred since under such combinations of conditions there exists the possibility of breakdown of the cellulose and/or lignin content of the ligno-cellulosic material which is not desirable. Such combinations of severe conditions may also cause degradation of the xylose produced to some extent. The hydrolysis step causes breakdown of hemicellulose molecules to produce xylans which are hydrolysed to add molecules of water and thereby produce xylose molecules. Any proteins or lipids which are released as a result of the hydrolysis go into solution. Lignin and cellulose are not affected and do not go into solution. These insoluble materials are separated in separation step (2). Any suitable separation method may be used in step (2), filtration being very suitable.

After separation of the insoluble residue containing cellulose and lignin, the aqueous xylose-containing medium is treated in removal step (3) to remove organic and/or ionic contaminents therefrom. The xylose-containing medium may be treated to remove organic or ionic contaminents in either order but preferably organic contaminents are removed first. The most significant organic contaminents are proteinaceous materials, lipids and coloured materials. The organic contaminents may be removed by any suitable method, e.g. treatment with reagents such as activated charcoal or polymeric absorbents. The treatment reagent may be contained in a bed through which the aqueous medium is passed or the reagent may be added to the medium in a tank. The most significant ionic contaminent is the acid used in hydrolysis step (1) but other inorganic contaminents may also be present. Removal of inorganic contaminents can be achieved by treatment with an electro-dialyser and/or a suitably ion-exchange resin, any conventional cationic or anionic ion-exchange resins being suitable. A combination of the electro-dialyser and the ion-exchange resin treatments can be used, the xylose-containing medium being subjected first to electro-dialyser treatment followed by passage through a bed of ion-exchange resin.

Suitable anionic ion-exchange resins for use in removal step (3) include resins formed from cross-linked polystyrene containing quaternary ammonium groups or substituted amines, polycondensation products of phenol and formaldehyde, polymerisation products of aromatic amines and formaldehyde guanidine-formaldehyde resins and polyamines. These anionic ion-exchange resins are commercially available as, for example, "AMBERLITE" (Registered Trade Mark) types IR-4B and IRA-400, "DOWEX" (Registered Trade Mark) types 1 and 2 and "PERMUTIT" (Registered Trade Mark) types S and D.R.

Suitable cationic ion exchange resins include polystyrene sulphonic acid type resins such as "AMBERLITE" IR-120 and "DOWEX" 50.

The xylose-containing medium passing to concentration step (4) is typically a solution containing 1% to 50% by weight xylose. In step (4) this solution is concentrated to a thick syrup having a water content in the range 20% to 40% by weight with the preferred range being 30% to 37% by weight. It is possible to operate the process using water contents below 30% by weight based on a decision to compromise between purity and yield. Below 20% by weight the syrup becomes too viscous for practical operation. Such a syrup is sufficiently concentrated to enable xylose to crystallise out when the syrup is mixed with ethanol in crystallization step (5). Concentration of the xylose-containing medium is carried out under conditions such that any significant degradation of xylose is avoided. Evaporation, particularly rotary or falling film evaporation, is preferred.

The syrup produced by concentration step (4) typically has a temperature in the range 10°C to 65°C, preferably in the range 10°C to 20°C, when it passes to crystallization step (5). In the crystallisation step, which is preferably carried out at ambient temperature, ethanol is added to the syrup. Suitably not more than 2 volumes of ethanol are added per volume of syrup. The addition of 0.5 to 2 volumes of ethanol per volume of syrup is preferred with addition of 1 volume ethanol to 1 volume of syrup being especially suitable. The ethanol/syrup mixture is allowed to stand for a period, suitably 10 to 18 hours, with occasional shaking to allow xylose to crystallize. It is possible to improve the crystallization process by seeding with xylose crystals. It is found that the ethanol will preferentially dissolve the hydrolysed materials which have passed through the various purification stages without dissolving a substantial amount of xylose which is sparingly soluble in ethanol is less soluble in this solvent than are other sugars. Thus the xylose which crystallizes has a high degree of purity. After crystallization of xylose the remaining aqueous liquor may be re-cycled, e.g. to step (3) and subjected to further extraction. In step (5) it is preferred to add ethanol to the syrup to avoid too great an amount of ethanol being present temporarily.

The process of the invention is advantageous in that xylose losses during the separation are reduced and a high proportion of the xylose present as xylan in the ligno-cellulosic material is extracted.

The invention is illustrated by the following Examples:

EXAMPLE 1

1 kg of wheat straw was treated with 9 l of a weak (2%) aqueous solution of sulphuric acid for 30 mins. at a temperature of 130°C. From the resultant mixture the insoluble residue comprising lignin and cellulose was separated by filtration. After separation of the insoluble residue, the aqueous medium was treated with activated charcoal (2.5 g charcoal per 100 mls medium) to remove organic contaminents. The medium was then filtered through a glass filter paper leaving contaminated activated charcoal behind. Thereafter the medium was neutralised with sodium hydroxide and electrodialized. After this treatment the medium at a temperature of 50°C was subjected to rotary evaporation to produce 256 g of a thick viscous syrup. Using a Silverson mixer this syrup was mixed with 200 ml ethanol and the resulting mixture was allowed to stand at room temperature (20°C) for 24 hours with occasional shaking. Seeding was found to be unnecessary in this instance. After 24 hours crystallization of xylose had taken place and 138 g of crystals were filtered off and washed with cooled ethanol before being air-dried. After analysis by high performance liquid chromatography the material was found to contain 93.4% xylose by weight, corresponding to a yield of 12.8% of xylose upon the weight of the straw.

EXAMPLE 2

A batch of hemicellulosic sugar syrup was extracted from chopped wheat straw in a 250 litre batch reactor. The hydrolysis reaction occurred in a weak aqueous solution of sulphuric acid held at 130°C for 30 mins. The resultant mixture was filtered and the filtrate treated with activated carbon followed by neutralisation with sodium hydroxide. The syrup was then electrodialysed to remove the bulk of any ionic contaminants. The liquor was then concentrated in a falling film evaporator to provide a supply of sugar syrup with the following analysis:

|  | % wt/wt | % of Total Sugars |
| --- | --- | --- |
| Xylose | 42.0 | 70.0 |
| Glucose | 9.6 | 16.0 |
| Arabinose | 5.3 | 8.8 |
| Galactose | 2.2 | 3.7 |
| Mannose | 0.9 | 1.5 |
| TOTAL SUGARS | 60.0 | 100.0 |

Aliquots of this bulk sample were taken and diluted or concentrated further to give samples with identical individual sugar ratios but different total sugar concentrations of 50.4%, 60%, 63.4% and 69.6% as analysed by HPLC.

Samples of 25 mls of the sugar syrup with the different above concentrations were taken and treated under a range of conditions to determine the percentage recovery of xylose crystals. Each 25 ml sample of syrup was mixed with either 25, 37.5 or 50 mls of ethanol at temperatures of ambient, 30°C or 50°C using a high speed Silverson mixer. The Silverson mixer was placed in the syrup and turned on. The ethanol was then added to the syrup over a period of 2 to 5.5 minutes. On completion of the addition of the required amount of ethanol, the mixer was left on for 1 minute further and then removed from the ethanol/sugar mixture. The mixture was then left to stand overnight before filtering off any crystals produced. The crystals were then dried and weighed. The percentage of total sugar precipitated was then calculated as the weight of dry crystals formed divided by the total mass of sugar in solution before the addition of ethanol.

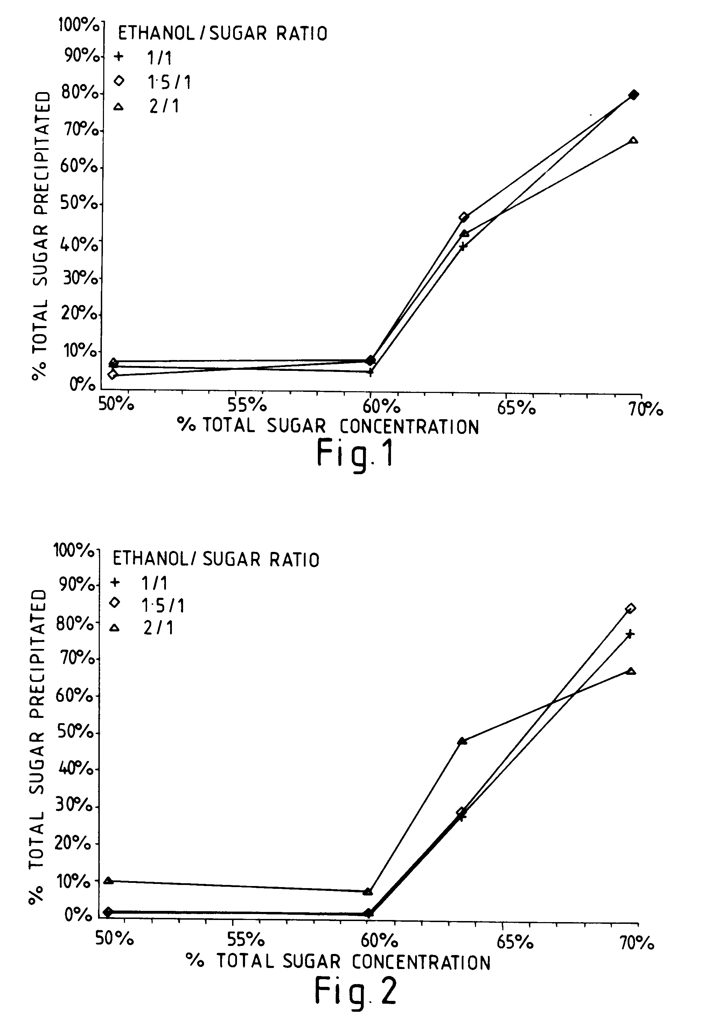

The results are shown in Figures 1, 2 and 3. These figures show the effect of % Sugar Concentration on the % Recovery of Sugar in the form of crystals for different ethanol/sugar ratios and temperatures. The figures clearly indicate that a critical syrup concentration (about 60 %) must be exceeded before the addition of ethanol if any reasonable recoveries of crystals are to be achieved. This critical concentration can be seen to be independent of ethanol/sugar ratio and temperature of the ranges investigated. There is also a clear increase in % sugar recovered as the % syrup concentration.increases above the critical concentration.

EXAMPLE 3

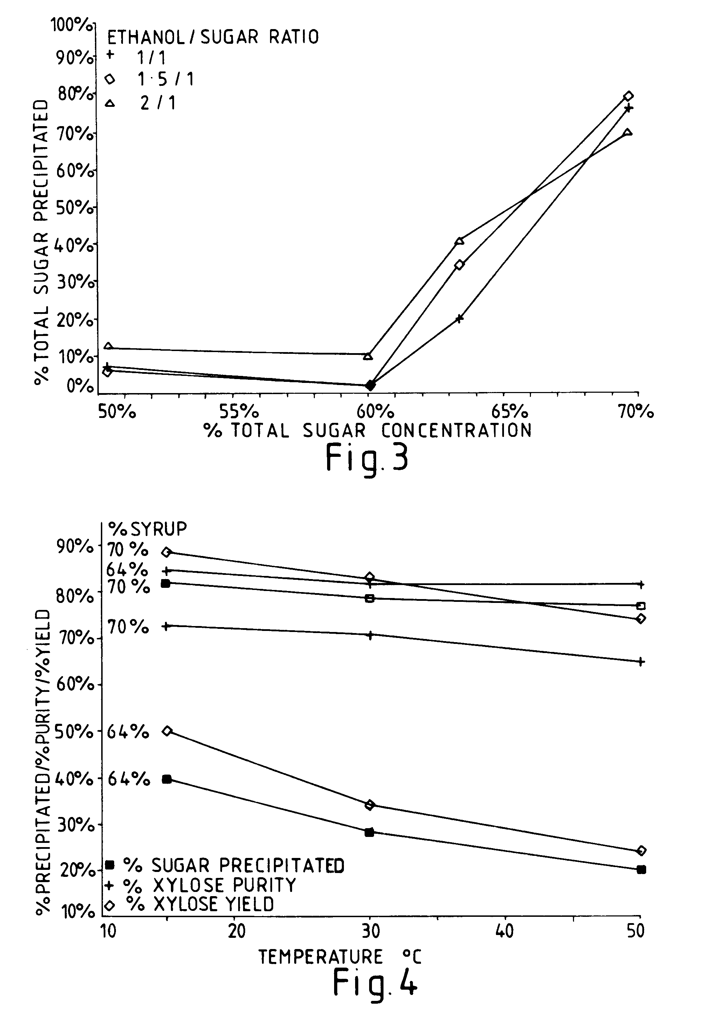

The dried sugar crystals obtained by the method described in Example 2 were analysed for xylose in order to calculate the % xylose purity of the crystals and thus the % yield of xylose in the crystal form. The results are shown in Figure 4 giving the effect of temperature on the % total sugar precipitated, % xylose crystal purity and % xylose yield for 2 syrup concentrations. Several conclusions can be drawn from this example:

1. The % yield of xylose increases with increasing syrup concentration but decreases with increasing temperature.

2. The xylose crystal purity increases with decreasing syrup concentration but remains roughly constant with temperature.

3. The total percentage of sugar precipitated increases with increasing syrup concentration but decreases with increasing temperature.

This example demonstrates that any decision must be made on the required purity and yield of xylose on the basis of economics as it is difficult to maximise both yield and purity simultaneously.

EXAMPLE 4

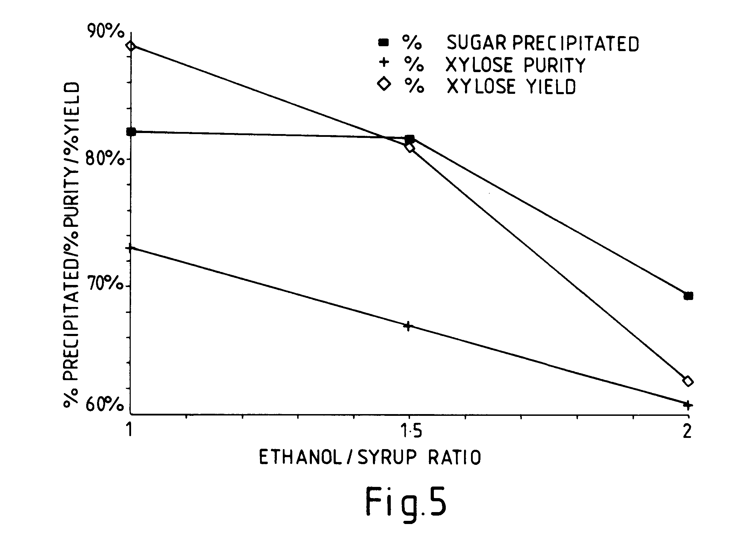

The method described in Example 2 was repeated to produce a syrup sample with a concentration of 70 %. 25 mls of syrup were mixed with differing quantities of ethanol at ambient temperature using a Silverson mixer and the method described in Example 2. The resulting crystals were dried and analysed for xylose to calculate the % sugar precipitated, % xylose purity and % xylose yield as described in Example 3. The results are shown in Figure 5 to give the effect of increasing the ethanol/syrup ratio on purity and yield. This example shows that although it is possible to obtain crystals with an ethanol/sugar ratio of 2:1, the effect of increasing the ethanol ratio is to reduce both purity and yield of the final crystals.

**Claims**

1. A process for the production of xylose from a lignocellulosic material comprising a xylan-containing hemicellulose, which comprises the steps of:-

   (1) hydrolysing the ligno-cellulosic material with an aqueous acid to produce an aqueous xylose-containing medium and an insoluble residue comprising lignin and cellulose;

   (2) separating the insoluble residue from the xylose-containing medium;

   (3) treating the xylose-containing medium to remove organic and/or ionic contaminants therefrom;

   (4) concentrating the xylose-containing medium to a syrup having a water content in the range 20% to 40% by weight; and

   (5) separating the xylose from the syrup by crystallization;

   characterised in that the separation of the xylose is carried out by mixing the syrup with ethanol to form an ethanolic solution and crystallizing xylose from the ethanolic solution.

2. A process according to claim 1 wherein the crystals produced in step (5) are washed with aqueous ethanol.

3. A process according to claim 1, or claim 2 wherein step (2) or step (3) is followed by a post hydrolysis step.

4. A process according to any one of the preceding claims wherein the aqueous acid used in step (1) is a solution containing 0.5% to 5% acid in water.

5. A process according to any one of the preceding claims wherein the reaction mixture is maintained at a temperature within the range 100° to 150°C during hydrolysis step (1).

6. A process according to any one of the preceding claims wherein the hydrolysis of step (1) is carried out for a period within the range 2 minutes to 2 hours.

7. A process according to any one of the preceding claims wherein in step (3) inorganic contaminents are removed by treatment with an electro-dialyser and/or a suitable ion-exchange resin.

8. A process according to any one of the preceding claims wherein in step (4) the xylose-containing medium is concentrated to a syrup having a water content in the range 30% to 37% by weight.

9. A process according to any one of the preceding claims wherein during step (5) ethanol is added to the syrup in a proportion in the range 0.5 to 2 volumes of ethanol per volume of syrup.

**10.** A process according to claim 9 wherein 1 volume of ethanol is added to 1 volume of syrup.

**Revendications**

**1.** Procédé pour la production de xylose à partir d'une matière lignocellulosique comprenant une hémicellulose contenant des xylanes, qui comprend les étapes suivantes :

(1) hydrolyse de la matière lignocellulosique avec un acide aqueux pour produire un milieu aqueux contenant du xylose et un résidu insoluble comprenant de la lignine et de la cellulose ;

(2) séparation du résidu insoluble du milieu contenant du xylose ;

(3) traitement du milieu contenant du xylose pour en éliminer des contaminants organiques et/ou ioniques ;

(4) concentration du milieu contenant du xylose en un syrop ayant une teneur en eau de l'ordre de 20 % à 40 % en poids ; et

(5) séparation du xylose à partir du syrop par cristallisation ;

caractérisé en ce que la séparation du xylose est effectuée par mélange du syrop avec de l'éthanol pour former une solution éthanolique et cristallisation du xylose à partir de la solution éthanolique.

**2.** Procédé suivant la revendication 1, caractérisé en ce que les cristaux produits dans l'étape (5) sont lavés avec de l'éthanol aqueux.

**3.** Procédé suivant la revendication 1 ou la revendication 2, caractérisé en ce que l'étape (2) ou l'étape (3) est suivie d'une étape de post-hydrolyse.

**4.** Procédé suivant l'une quelconque des revendications précédentes, caractérisé en ce que l'acide aqueux utilisé dans l'étape (1) est une solution contenant 0,5 % à 5 % d'acide dans l'eau.

**5.** Procédé suivant l'une quelconque des revendications précédentes, caractérisé en ce que le mélange réactionnel est maintenu à une température de l'ordre de 100 à 150°C pendant l'étape d'hydrolyse (1).

**6.** Procédé suivant l'une quelconque des revendications précécentes, caractérisé en ce que l'hydrolyse de l'étape (1) est effectuée pendant une période de l'ordre de 2 minutes à 2 heures.

**7.** Procédé suivant l'une quelconque des revendications précédentes, caractérisé en ce que dans l'étape (3), des contaminants inorganiques sont éliminés par traitement avec un électro-dialyseur et/ou une résine échangeuse d'ion convenable.

**8.** Procédé suivant l'une quelconque des revendications précédentes, caractérisé en ce que dans l'étape (4), le milieu contenant du xylose est concentré en un syrop ayant une teneur en eau de l'ordre de 30 % à 37 % en poids.

**9.** Procédé suivant l'une quelconque des revendications précédentes, caractérisé en ce que pendant l'étape (5), de l'éthanol est ajouté au syrop en une proportion de l'ordre de 0,5 à 2 volumes d'éthanol par volume de syrop.

**10.** Procédé suivant la revendication 9, caractérisé en ce qu'un volume d'éthanol est ajouté à un volume de syrop.

**Patentansprüche**

**1.** Verfahren zur Herstellung von Xylose aus einem Ligno-Cellulosematerial, das eine Xylan-enthaltende Hemicellulose umfaßt, **dadurch gekennzeichnet,** daß es folgende Schritte umfaßt:

(1) Hydrolyse des Ligno-Cellulosematerials mit einer wäßrigen Säure zur Herstellung eines wäßrigen Xylose-enthaltenden Mediums und eines unlöslichen Rückstandes, der Lignin und Cellulose umfaßt,

(2) Trennung des unlöslichen Rückstandes von dem Xylose-enthaltenden Medium,

(3) Behandlung des Xylose-enthaltenden Mediums zur Entfernung von organischen und/oder ionischen Verunreinigungen daraus,

(4) Konzentrierung des Xylose-enthaltenden Mediums zu einem Sirup, mit einem Wassergehalt im Bereich von 20 bis 40 Gew.-% und

6

(5) Trennung der Xylose von dem Sirup durch Kristallisation,
**dadurch gekennzeichnet,** daß die Trennung der Xylose durch Vermischung des Sirups mit Ethanol unter Bildung einer ethanolischen Lösung und Kristallisation der Xylose aus der ethanolischen Lösung ausgeführt wird.

2. Verfahren nach Anspruch 1, **dadurch gekennzeichnet,** daß die in Schritt (5) hergestellten Kristalle mit wäßrigem Ethanol gewaschen werden.

3. Verfahren nach Anspruch 1 oder 2, **dadurch gekennzeichnet,** daß auf Schritt (2) oder schritt (3) ein Nachhydrolyseschritt folgt.

4. Verfahren nach einem der vorhergehenden Ansprüche, **dadurch gekennzeichnet,** daß die in Schritt (1) verwendete wäßrige Säure eine Lösung ist, die 0,5 % bis 5 % Säure in Wasser enthält.

5. Verfahren nach einem der vorhergehenden Ansprüche, **dadurch gekennzeichnet,** daß die Reaktionsmischung bei einer Temperatur im Bereich von 100°C bis 150°C während des Hydrolyseschritts (1) gehalten wird.

6. Verfahren nach einem der vorhergehenden Ansprüche, **dadurch gekennzeichnet,** daß der Hydrolyseschritt (1) während eines Zeitraums innerhalb des Bereichs von 2 Minuten bis 2 Stunden ausgeführt wird.

7. Verfahren nach einem der vorhergehenden Ansprüche, **dadurch gekennzeichnet,** daß in Schritt (3) anorganische Verunreinigungen durch Behandlung mit einem Elektrodialysierapparat und/oder einem geeigneten Ionenaustauscherharz entfernt werden.

8. Verfahren nach einem der vorhergehenden Ansprüche, **dadurch gekennzeichnet,** daß in Schritt (4) das Xylose-enthaltende Medium zu einem Sirup mit einem Wassergehalt im Bereich von 30 bis 37 Gew.-% konzentriert wird.

9. Verfahren nach einem der vorhergehenden Ansprüche, **dadurch gekennzeichnet,** daß während Schritt (5) dem Sirup Ethanol in einem Verhältnis im Bereich von 0,5 bis 2 Volumina Ethanol pro Volumen Sirup zugegeben wird.

10. Verfahren nach Anspruch 9, **dadurch gekennzeichnet,** daß ein Volumen Ethanol zu einem Volumen Sirup zugegeben wird.

Fig.1

Fig.2

Fig.3

Fig.4

Fig.5